# EUROPEAN PATENT APPLICATION

(11) **EP 4 516 772 A1**
(43) Date of publication of application: **05.03.2025**
(21) Application number: 23795824.4
(22) Date of filing: 20.01.2023
(51) Int. Cl.: C07C 51/42, C07C 57/07, B01J 19/00

(54) **DEVICE FOR HANDLING EASILY POLYMERIZABLE SUBSTANCE**

(30) Priority: 27.04.2022 JP 2022073001
(71) Applicant: Nippon Shokubai Co., Ltd., Osaka-shi, Osaka 541-0043 (JP)
(72) Inventor: KIMURA, Yuki, Himeji-shi, Hyogo 671-1282 (JP); KIMURA, Hayato, Himeji-shi, Hyogo 671-1282 (JP)
(74) Representative: Schön, Christoph
(86) International application number: PCT/JP2023/001662
(87) International publication number: WO 2023/210075

(57) **Abstract**

[Problem] To prevent or suppress generation of polymers in the internal space of a protrusion unit.

[Solution] A handling device 100 includes a flow unit 10, a protrusion unit 30, a spray unit 40, and a grid 50. The spray unit is configured to be able to be disposed in an internal space 31 of the protrusion unit and be able to spray liquid onto the inner wall surface of the protrusion unit with the spray unit disposed in the internal space. The grid is disposed at the boundary of a flow path 11 of the flow unit and the internal space of the protrusion unit to prevent irregular packing F packed in a packed bed 17 from moving to the internal space.

## Description

### TECHNICAL FIELD

The present invention relates to a handling device for easily polymerizable substance.

### BACKGROUND ART

It is well known that an easily polymerizable substance such as (meta)acrylic acid is extremely prone to polymerization and often undergoes polymerization in its process for production, which forces device stoppage. Measures for this include use of a polymerization inhibitor such as hydroquinone or phenothiazine and introduction of a molecular oxygen-containing gas.

In a process for production of an easily polymerizable substance such as (meta) acrylic acid, a packed column is used for its capture and purification in some cases, and polymer formation is inevitable even in the packed column. The easily polymerizable substance such as (meta)acrylic acid is likely to stagnate at a stagnation place such as a maintenance hole in the packed column, where a polymerization inhibitor is consumed, and polymers are generated when the polymerization inhibitor eventually becomes short.

In this manner, the above-described easily polymerizable substance such as (meta)acrylic acid is a compound that is extremely prone to polymerization, and thus various technologies for preventing polymerization in a handling device for easily polymerizable substance such as a packed column described above have been proposed. For example, Patent Literature 1 discloses a technology of preventing polymerization attributable to stagnation by spraying, on the inner wall surface of a device such as a maintenance hole at a stagnation place, liquid that would accumulate on a tray inside a column that performs distillation or the like.

### Citation List

### Patent Literature

Patent Literature 1: US 2017/0014730 A1

### SUMMARY OF INVENTION

### Technical Problem

Packing such as irregular packing can be introduced inside a packed column as a handling device for easily polymerizable substance described above. An opening region where a maintenance hole or the like can be installed is provided on a side of a packed bed inside a flow path in the handling device, and an internal space is provided between the opening region and the flow path. The inventors have been investigating a technology of preventing or suppressing the occurrence of a phenomenon that an easily polymerizable substance such as (meta)acrylic acid stagnates and polymers are generated in the internal space communicating with the opening region where a maintenance hole or the like can be installed in the above-described handling device.

### Means for Solving Problem

An aspect of the present invention is a handling device for easily polymerizable substance. The handling device includes a flow unit, a protrusion unit, a spray unit, and a grid. The flow unit forms a flow path through which an easily polymerizable substance-containing material flows and in which a support member with which irregular packing can be packed is installed. The protrusion unit is provided on a side of a packed bed packed with the irregular packing in the flow unit so as to protrude outward in a direction intersecting a flow direction of the easily polymerizable substance-containing material, and forms an internal space in a protruding site and an opening region that leads to the internal space and on which a lid that separates the internal space from the outside can be installed. The spray unit is configured to be able to be disposed in the internal space of the protrusion unit and be able to spray liquid onto the inner wall surface of the protrusion unit with the spray unit disposed in the internal space. The grid is configured to be disposed at the boundary of the flow path and the internal space to prevent the irregular packing packed in the packed bed from moving to the internal space.

### Advantageous Effect of the Invention

With a handling device for easily polymerizable substance according to an aspect of the present invention, it is possible to prevent or suppress generation of polymers in the internal space of a protrusion unit.

### BRIEF DESCRIPTION OF DRAWINGS

Fig. 1 is a schematic view illustrating a handling device for easily polymerizable substance according to an embodiment of the present invention.
Fig. 2 is a diagram illustrating a flow path of a flow unit and the internal space of a protrusion unit in the handling device according to Fig. 1.
Fig. 3 is a diagram viewing the handling device according to Fig. 2 from a direction in which a lid is installed on an opening region of the protrusion unit.
Fig. 4 is a cross-sectional view illustrating a state in which a spray unit is disposed in the internal space of the protrusion unit in the handling device in Fig. 1.
Fig. 5 is a diagram of a modification of the spray unit, corresponding to Fig. 4.
Fig. 6 is a diagram of a modification of the spray unit, corresponding to Fig. 2.
Fig. 7 is a diagram of a modification of the spray unit, corresponding to Fig. 2.

### DESCRIPTION OF EMBODIMENTS

An embodiment of the present invention will be described below in detail with reference to the accompanying drawings. The embodiment described herein is exemplarily described to materialize the technical idea of the present invention and does not limit the present invention. Other feasible embodiments, examples, operation technologies, and the like that could be thought of by the skilled person in the art or the like without departing from the scope of the present invention are all included in the range and scope of the present invention, and also included in the range of the invention written in the claims and its equivalents.

In the accompanying diagrams of the present specification, scales, ratios of longitudinal and transverse dimensions, shapes, and the like may be changed from those in reality and schematically illustrated as appropriate for the sake of convenience of illustration and ease of understanding, but are merely examples and do not limit interpretation of the present invention.

In the following description, ordinals such as "first" and "second" are used for the sake of convenience and do not define any order unless otherwise stated.

Note that an "easily polymerizable substance" in the following description means a radical polymerizable monomer that is polymerizable due to temperature, pressure, contact mixing, light, or the like. Examples of such an easily polymerizable substance include unsaturated carboxylic acids such as acrylic acid, methacrylic acid, fumaric acid, maleic acid, and maleic anhydride, and their esters. Note that acrylic acid and/or methacrylic acid are referred to as "(meta) acrylic acid" in some cases.

Examples of hydroxyl-containing compounds capable of forming the above-described unsaturated carboxylic acids and esters include aliphatic alcohols or alicyclic alcohols with 1 to 12 carbon atoms. Examples of such hydroxyl-containing compounds include monohydric alcohols such as methanol, ethanol, n-butanol, iso butanol, sec-butanol, t-butanol, 1-pentanol, 2-pentanol, 3-pentanol, cyclopentanol, 1-hexanol, 2-hexanol, 3-hexanol, cyclohexanol, 1-heptanol, 2-heptanol, 3-heptanol, 1-octanol, iso octanol, 2-ethyl hexanol, isononyl alcohol, and lauryl alcohol; and polyhydric alcohols such as ethylene glycol and 1,2-propanediol, and these may be straight-chain or branched-chain.

The easily polymerizable substance is preferably acrylic acid, methacrylic acid, acrylic ester, or methacrylic ester, more preferably, acrylic acid, methacrylic acid, methyl acrylate, ethyl acrylate, butyl acrylate, 2-ethylhexyl acrylate, 2-hydroxyethyl acrylate, 2-hydroxypropyl acrylate, methyl methacrylate, ethyl methacrylate, butyl methacrylate, 2-ethylhexyl methacrylate, 2-hydroxyethyl methacrylate, or 2-hydroxypropyl methacrylate, further more preferably, acrylic acid or methacrylic acid.

"Handling" in the present application specification means at least one operation of reaction, distillation, extraction, absorption, storage, heat exchange, and the like, which are necessary for manufacturing the easily polymerizable substance. Accordingly, a "device" in the present application specification means a reactor, a distillation column, an extraction column, an absorption column, a heat exchanger, a tank, and the like, which are used to perform the above-described handling such as reaction, distillation, extraction, absorption, storage, and heat exchange. Note that a column packed with packing in a flow path of a distillation column or the like is referred to as a packed column.

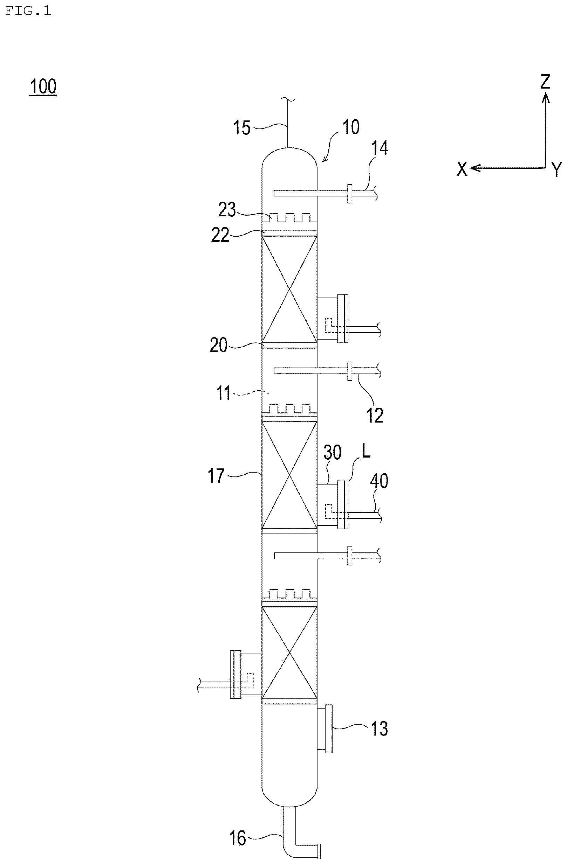

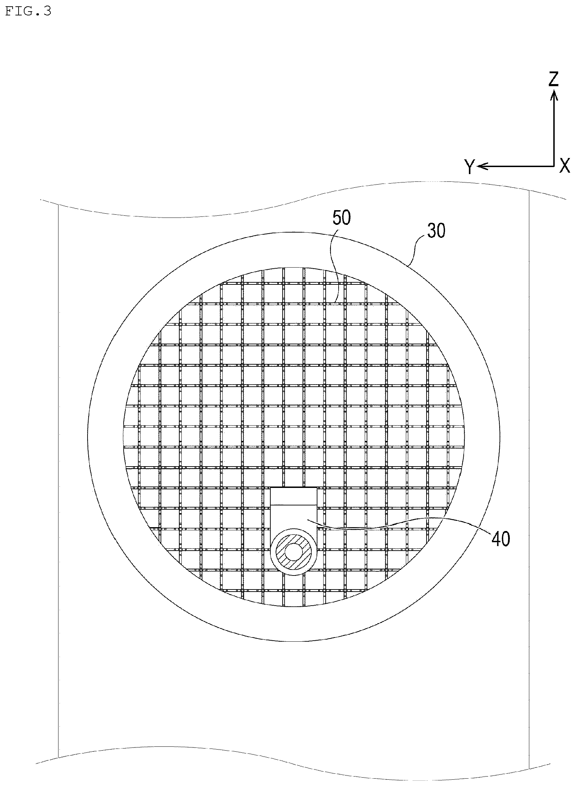

Figs. 1 to 7 are diagrams for description of a handling device 100. The handling device 100 includes a flow unit 10, a support member 20, a protrusion unit 30, a spray unit 40, and a grid 50. In addition, the handling device 100 includes sites (13 to 16) where gas or liquid flows. Note that a coordinate system is illustrated in the drawings for the sake of descriptive convenience. X corresponds to an insertion direction of the spray unit 40 described later and is referred to as a first horizontal direction X. Y is a direction orthogonal to the first horizontal direction X in the horizontal direction and referred to as a second horizontal direction Y. Z is a direction orthogonal to the first horizontal direction X and the second horizontal direction Y and referred to as a height direction Z.

The flow unit 10 is configured to form a flow path 11 through which an easily polymerizable substance-containing material in the form of gas or liquid flows and so that the support member 20 with which irregular packing F can be packed is installed on the flow path 11. The flow unit 10 is configured to form a flow path through which gas or liquid flows in the handling device 100. The flow unit 10 is configured to extend in the height direction Z in the present embodiment.

The flow unit 10 is configured to be provided with a liquid inlet 12, a gas inlet 13, a gas outflow port 15, and a liquid outflow port 16 as illustrated in Fig. 1. An inlet 14 for reflux liquid obtained by cooling (condensing) gas discharged from the gas outflow port 15 is provided at an upper part of the handling device (illustration of a flow path for cooling gas is omitted). The gas inlet 13 is configured to be provided at a part lower than the gas outflow port 15, and the liquid inlet 12 is configured to be provided at a part upper than the liquid outflow port 16. The gas inlet 13 is configured to be disposed at a part lower than the liquid inlet 12 in the height direction Z.

The support member 20 is configured to be able to form a site where the irregular packing F is placed in the flow path 11 of the fluid formed by the flow unit 10. The site where the irregular packing F is packed by the support member 20 in the flow unit 10 may be referred to as a packed bed 17. The support member 20 may be a conventionally well-known support member such as a wire mesh structured packing support member or an arch-shaped folded plate structured packing support member. For example, the arch-shaped folded plate structured packing support member is illustrated in Figs. 18 to 51 of Perry's "Chemical Engineers' Handbook, 5th Edition".

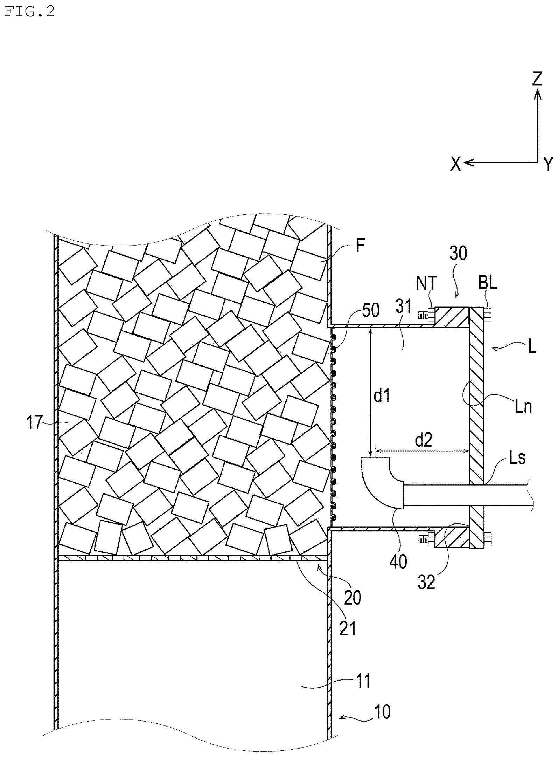

The support member 20 is configured to be provided with a plurality of holes 21 as illustrated in Fig. 2. With this configuration, gas flowing in from the gas inlet 13 across the support member 20 and liquid flowing in from the liquid inlet 12 can flow in the height direction Z in the internal space of the flow unit 10.

Note that, as illustrated in Fig. 1, a packing restraint 22 for restraining the irregular packing F is provided on the upper surface of the packed bed 17, and a liquid distributor 23 is further provided thereon. With this configuration, liquid introduced from the liquid inlet 12 and the reflux liquid inlet 14 is evenly distributed over the entire upper surface of the packed bed 17 by passing through the liquid distributor 23. The packing restraint 22 is a member similar to the support member 20. The liquid distributor 23 may use, for example, an open trough type, a pipe orifice type, a fishbone type.

The irregular packing F may use, but is not particularly limited, by way of example, Raschig rings, Pall rings, Intalox saddles, Tellerettes, Cascade mini-rings, or IMTP.

As illustrated in Fig. 2, the protrusion unit 30 is disposed on a side of the packed bed 17 in which the irregular packing F is disposed in the flow unit 10. The protrusion unit 30 is provided to protrude outward in the horizontal direction intersecting the flow direction of the easily polymerizable substance-containing material. The protrusion unit 30 is provided with an internal space 31 and an opening region 32 as illustrated in Fig. 2 or the like. The internal space 31 is configured to be able to communicate with the flow path 11 of the flow unit 10 through the grid 50. The opening region 32 is configured to leads to the internal space 31 of the protrusion unit 30, and be able to install a lid L that separates the internal space 31 from the outside. The form of installation of the lid L on the opening region 32 is not particularly limited, but may be, for example, coupling by davits or hinges.

The opening region 32 can be formed in a size (maintenance hole) with which a worker can enter through the lid L, a size (hand hole) with which the worker can insert a hand through the opening region 32, or a size with which a person can view the flow path of the flow unit 10 through the opening region 32. The lid L can be formed to be integrated by well-known bolts BL and nuts NT or the like. The protrusion unit 30 is constituted by the side surface of a cylindrical shape extending in the first horizontal direction X in the present embodiment.

The spray unit 40 is configured to be able to be inserted into the internal space 31 of the protrusion unit 30 through the lid L installed on the opening region 32 and be able to spray liquid onto the inner wall surface of the protrusion unit 30 with the spray unit 40 disposed in the internal space 31. The spray unit 40 is configured such that the liquid sprayed onto the inner wall surface of the protrusion unit 30 is configured to move along the inner wall surface of the protrusion unit 30 by gravitational force and to wet the entire inner wall surface of the protrusion unit 30. The entire inner wall surface of the protrusion unit 30 herein includes not only a tubular inner side surface forming the internal space 31 of the protrusion unit 30 but also an inner side surface Ln of the lid L. Spray patterns (spray shapes) of the spray unit 40 include a flat spray type, a full cone spray type, and a hollow cone spray type and the like.

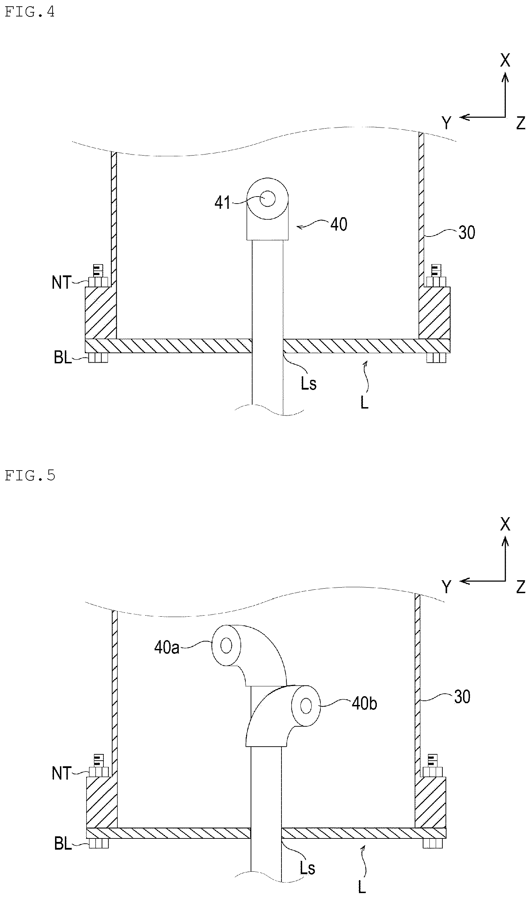

The spray unit 40 is configured to be able to be inserted into the internal space 31 through an insertion hole Ls of the lid L that closes the opening region 32 from the outside as illustrated in Fig. 4 or the like. The spray unit 40 in the present embodiment is configured so that liquid is sprayed upward, in particular, in the state of installation in the internal space 31 of the protrusion unit 30. In Fig. 4, the spray unit 40 is provided with one liquid spray port 41. The spray port 41 of the spray unit 40 may be disposed to face upward in the height direction Z (refer to Figs. 2 and 4). In a case where the spray unit 40 is not inserted into a site of the lid L where the spray unit 40 is to be inserted, the site may be blocked by a hole filling cap or plug (not illustrated).

The grid 50 is configured to be disposed at the boundary of the flow path 11 of the flow unit 10 and the internal space 31 to prevent the irregular packing F disposed in the packed bed 17 from moving toward the internal space 31. The grid 50 is configured to include a wire mesh with numerous gaps in a predetermined size. With this configuration, the worker can check the internal space 31 of the protrusion unit 30 and the flow path 11 of the flow unit 10 through the opening region 32 when the lid L installed on the opening region 32 is opened. The size of the wire mesh of the grid 50 is not particularly limited but may be any size with which the irregular packing F can be prevented from entering the internal space 31, and can be formed from 10 mm to 70 mm, for example.

The meshes of the grid 50 are formed to have a size equal to or smaller than the size (minimum dimension) of the irregular packing F to prevent the irregular packing F from entering the internal space 31 of the protrusion unit 30 from the flow path 11 through the meshes. The "minimum dimension" here means the smallest value among dimensions such as length, width, height, and diameter that specify the shape of the irregular packing F in the specifications of the irregular packing F. The grid 50 in the present embodiment is configured to be detachable from the flow unit 10 and the protrusion unit 30 in the handling device 100. The grid 50 may be made of material such as metal and is preferably material equivalent to the irregular packing F.

As described above, the handling device 100 in the present embodiment includes the flow unit 10, the protrusion unit 30, the spray unit 40, and the grid 50. The flow unit 10 forms the flow path 11 through which the easily polymerizable substance-containing material flows and in which the support member 20 with which the irregular packing F can be packed is installed. The protrusion unit 30 is provided on a side of the packed bed 17 packed with the irregular packing F in the flow unit 10 so as to protrude outward in the direction intersecting the flow direction of the easily polymerizable substance. The protrusion unit 30 forms the internal space 31 at a protruding site and the opening region 32 that leads to the internal space 31 and on which the lid L that separates the internal space 31 from the outside can be installed. The spray unit 40 is configured to be able to be disposed in the internal space 31 of the protrusion unit 30 and be able to spray liquid onto the inner wall surface of the protrusion unit 30 with the spray unit disposed in the internal space 31. The grid 50 is disposed at the boundary of the flow path 11 and the internal space 31 to prevent the irregular packing F packed in the packed bed 17 from moving to the internal space 31.

Irregular packing is often used in the handling device 100 used in distillation operation, absorption operation, extraction operation, or the like in a process for production of the easily polymerizable substance. Polymers are likely to be generated due to stagnation of the easily polymerizable substance (in particular, stagnation of liquid condensed from vapor phase) at a protrusion unit directly coupled to a packed bed of the irregular packing. Once polymers are generated at the protrusion unit, the polymers act as nuclei and gradually grow and accumulate, causing polymerization in the packed bed, increasing pressure difference of the packed bed, and eventually blocking the inside of the device. This hinders continuous operation of the handling device. Furthermore, removal of the generated polymers is extremely difficult and the cost of removal is significant.

In contrast, the handling device 100 is provided with the grid 50 as described above to prevent the irregular packing F from entering the internal space 31 in which the spray unit 40 is installed, thereby ensuring the space. Moreover, liquid is sprayed upward from the position of the spray unit 40 in a state in which the irregular packing F is prevented from entering the internal space 31 by the grid 50, and thus the entire inner wall surface of the protrusion unit 30 can be wetted with the liquid. Accordingly, blockage in the device due to polymers can be prevented. Further, since the handling device 100 includes the grid 50 and the spray unit 40, continuous operation of the device is promoted and the cost of polymer removal can be suppressed. In addition, since the grid 50 is used as a component for preventing the irregular packing F from entering the internal space 31, the worker who opens the lid L can inspect the status of the internal space 31 and the flow path 11 positioned farther than the internal space 31, through the opening region 32.

Liquid sprayed through the spray unit 40 is not particularly limited, but may be any liquid containing a polymerization inhibitor that inhibits polymerization of the easily polymerizable substance and use, for example, liquid discharged from a column bottom part of the packed column, reflux liquid introduced at a column top part thereof, or liquid obtained from another process.

The meshes of the grid 50 are configured to have a size equal to or smaller than the minimum dimension of the irregular packing F. With this configuration, the irregular packing F can be prevented from entering the internal space 31 through the meshes of the grid 50.

The spray unit 40 sprays liquid upward in the internal space 31 of the protrusion unit 30. With this configuration, the liquid adheres to the inner wall surface of an upper part of the internal space 31 and then spreads out downward along the inner wall surface of the internal space 31 by gravitational force. As a result, the entire inner wall surface forming the internal space 31 of the protrusion unit 30 can be wetted with the liquid.

The grid 50 is configured to be detachable from the flow unit 10. With this configuration, it can be easy for the worker to perform maintenance of, for example, the flow path 11 of the flow unit 10 in the handling device 100.

The spray unit 40 is configured to be able to be inserted into the internal space 31 through the lid L that closes the opening region 32 from the outside. With this configuration, the spray unit 40 can be inserted into the internal space 31 without removing the lid L to spray liquid to the internal space 31 of the protrusion unit 30, thereby preventing or suppressing generation of polymers due to the easily polymerizable substance.

### [Example]

The following describes an experiment performed on the wetting state of a liquid containing polymerization inhibitor and the polymerization state of the easily polymerizable substance at the protrusion unit 30 in the handling device 100 according to the present embodiment. In the present experiment, acrylic acid gas was absorbed into water in a packed column corresponding to the handling device 100 packed with the irregular packing F. In the present experiment, the wetting state of the inner wall surface of the protrusion unit with the polymerization inhibitor-containing liquid was checked for one example and three comparative examples as described below.

In Example 1, the grid 50 was installed at the boundary between the protrusion unit 30 and the flow unit 10 in the handling device 100. Then, the spray unit 40 was inserted into the internal space 31 of the protrusion unit 30 through the insertion hole of the lid L and sprayed the polymerization inhibitor-containing liquid upward. Hereinafter, for the sake of simplicity, any component of the handling device 100 common to the example is denoted by the same reference sign in description of the comparative examples. The grid 50 was a lattice with a hole shape of 50 mm in height and width. A flat wide angle spray nozzle 3/8EX-6200W (pipe diameter: 20 A) manufactured by Niikura Kogyo Co., Ltd. was used for the spray unit 40.

The configuration of Comparative Example 1 is the same as that of the example except that no grid 50 is installed at the boundary of the flow unit 10 and the protrusion unit 30 in the packed column corresponding to the handling device 100. In the packed column according to Comparative Example 2, the grid 50 is installed at the boundary of the flow unit 10 and the protrusion unit 30 like Example 1, and no spray unit 40 was disposed at a site corresponding to the internal space 31 of the protrusion unit 30. In the packed column according to Comparative Example 3, no grid 50 is installed at the boundary of the flow unit 10 and the protrusion unit 30 unlike Example 1 and no spray unit 40 was disposed in the internal space 31 of the protrusion unit 30.

Conditions common to Example 1 and the three comparative examples described above are that the diameter of the opening region 32 of the protrusion unit 30 is 500 mm and the distance from the inner side surface Ln of the lid L to the grid 50 is 80 mm. The position (insertion position in the height direction Z: distance d1 in Fig. 2) of the spray port 41 of the spray unit 40 in Example 1 is 340 mm from the upper end of the inner wall surface of the protrusion unit 30, and distance d2 (refer to Fig. 2) in the first horizontal direction X from the inner side surface Ln of the lid L is 70 mm. The spray port 41 of the spray unit 40 was configured to be oriented in the height direction Z. In the irregular packing F, Pall ring was used.

As confirmed in Comparative Example 1, the irregular packing F entered the internal space 31 of the protrusion unit 30 from the flow unit 10 and, because the irregular packing F was present in the spray range of the spray unit 40, the entire inner wall surface of the protrusion unit 30 could not be wetted with liquid. As a result, an acrylic acid aqueous solution stagnated on the inner wall surface of the protrusion unit 30, and polymers were generated at a stagnation part where the polymerization inhibitor was consumed. Due to this, polymers were generated in the packed bed 17 and a pressure loss in the packed column increased.

In Comparative Example 2, the inner wall surface of the protrusion unit 30 could not be wetted with liquid since the spray unit 40 was not used. As a result, an acrylic acid aqueous solution stagnated on the inner wall surface of the protrusion unit 30, and polymers were generated at a stagnation part where the polymerization inhibitor was consumed. Due to this, polymers were generated in the packed bed 17 and a pressure loss in the packed column increased.

In Comparative Example 3, the irregular packing F entered the internal space 31 of the protrusion unit 30 since the grid 50 was not used, and the inner wall surface of the protrusion unit 30 could not be wetted with liquid since the spray unit 40 was not used. As a result, an acrylic acid aqueous solution stagnated on the inner wall surface of the protrusion unit 30, and polymers were generated at a stagnation part where the polymerization inhibitor was consumed. Due to this, polymers were generated in the packed bed 17 and a pressure loss in the packed column increased.

In contrast, in the handling device 100 according to Example 1, the irregular packing F did not enter the internal space 31 of the protrusion unit 30 like Comparative Examples 1 to 3, and stagnation of an acrylic acid aqueous solution on the inner wall surface of the protrusion unit 30 was prevented by wetting the inner wall surface thereof with the liquid containing the polymerization inhibitor. As a result, polymers were not generated, and the device was stably operated for one year.

In addition, the status (wetting state) of the inner wall surface of the protrusion unit 30 was checked in a case where liquid was sprayed from the spray port 41 of the spray unit 40 in the handling device 100 according to Example 1 at the flow rate in six kinds of 0.3, 0.4, 0.5, 0.6, 0.7, and 1.0 m³/h. As a result, the flow rate of 0.3 m³/h was too low to wet the entire inner wall surface of the protrusion unit 30 with liquid. In particular, it was observed that part of the inner side surface Ln of the lid L was not wetted.

In the case of the flow rate of 0.7 or 1.0 m³/h, momentum was too large to uniformly wet the entire inner wall surface of the protrusion unit 30. Excessive flow rate setting is not preferable because physical corrosion due to water flow is a concern.

In the case of the flow rate of 0.4, 0.5, or 0.6 m³/h, the flow rate was not too low unlike 0.3 m³/h and too large liquid momentum was not observed unlike 0.7 m³/h and the like. Thus, it was checked that it is preferable to spray liquid at a flow rate in the range of 0.4 to 0.6 m³/h (0.5m³/h, in particular) to evenly wet the entire inner wall surface of the protrusion unit 30 in the handling device 100.

Note that the present invention is not limited to only the above-described embodiment, and various changes may be made to the present invention within the scope of the claims. Figs. 5 to 7 are diagrams illustrating a modification of the spray unit 40 in the handling device 100, Fig. 5 corresponding to Fig. 4, Figs. 6 and 7 corresponding to Fig. 2.

In the above description, it is described that the spray unit 40 is provided with one nozzle such that the spray port 41 of the spray unit 40 faces upward in the height direction Z. However, the orientation of the nozzle and the number thereof are not limited to the above description as long as liquid can be wholly sprayed over the internal space 31 of the protrusion unit 30. Unlike in the above description, a plurality of nozzles such as spray units 40a and 40b may be provided. Moreover, the spray port of each spray unit does not necessarily need to be oriented in the height direction Z, and therefore, the spray port thereof may be configured to be oriented, for example, at a tilt from the height direction Z about a rotational axis in the first horizontal direction X (insertion direction of the spray unit) as illustrated with the spray units 40a and 40b in Fig. 5.

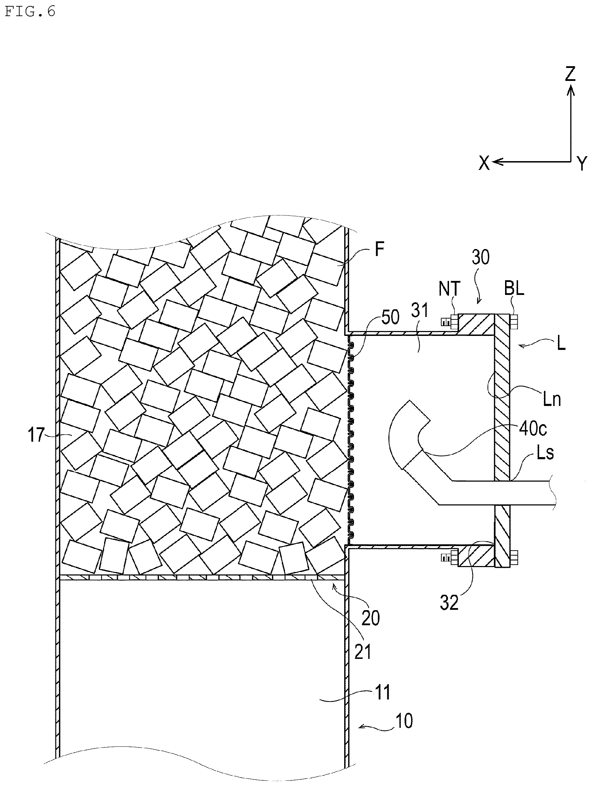

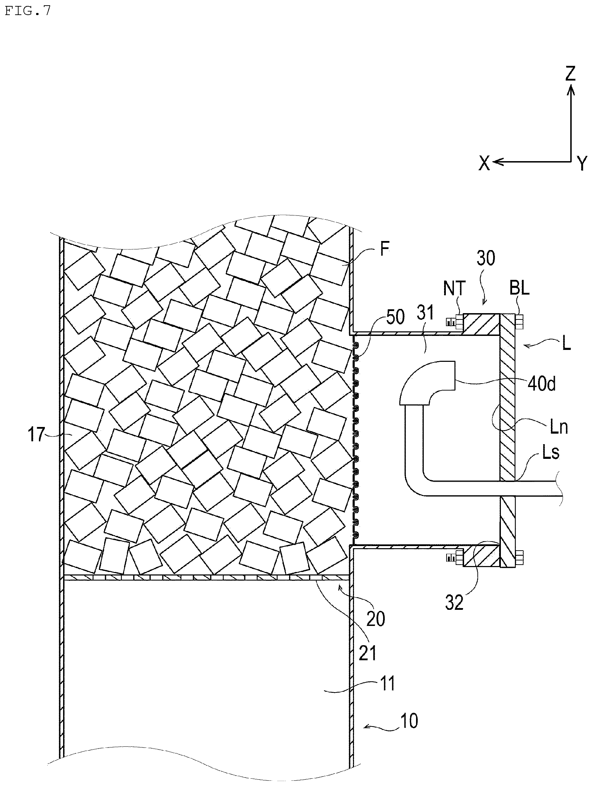

In the above description, the spray port 41 of the spray unit 40 is oriented upward from the nozzle along the height direction Z when viewed from the second horizontal direction Y, which is orthogonal to the first horizontal direction X and the height direction Z, as illustrated in Fig. 2. However, the orientation of the spray port of the spray unit is not limited to Figs. 2 to 4 or the like as long as the entire inner wall surface of the protrusion unit 30 can be evenly wetted. Unlike the above description, the spray port of a spray unit 40c may be configured to be oriented obliquely upward relative to the second horizontal direction Y so as to face the lid L as illustrated in Fig. 6. In the present specification, "upward" means a configuration in which the orientation of the spray port of the spray unit has at least a component in the height direction Z in vector decomposition, and includes not only a case where the spray port is oriented in the height direction Z as illustrated in Fig. 2 but also a case where the spray port is oriented obliquely upward as illustrated in Fig. 6. As illustrated in Fig. 7, the spray port of a spray unit 40d may be configured to be oriented toward a side by, for example, curving its intermediate pipe toward the lid L.

In the above description, the grid 50 is freely attachable to and detachable from the flow unit 10 and the protrusion unit 30. However, as long as the irregular packing F packed in the packed bed of the flow unit 10 can be prevented from entering the internal space 31, a case in which the grid 50 is not freely attachable and detachable, but is integrally joined with at least one of the flow unit 10 and the protrusion unit 30 by welding or the like is also included in an embodiment.

In the above description, the protrusion unit 30 is formed in a shape such as the side surface of a cylindrical shape. However, the shape of the protrusion unit 30 is not limited to the shape of the side surface of a cylindrical shape. In addition to the shape of the side surface of a cylindrical shape, the shape of the protrusion unit may be configured to be provided with a slant face tilted toward the flow unit 10 so that liquid stagnated in the protrusion unit 30 flows down to the flow unit 10, or may be tapered toward the lid L from the flow unit 10.

In the above description, the irregular packing F is packed in the packed bed 17. However, the present invention is not limited thereto, but packing other than the irregular packing may be packed in the packed bed.

The present invention encompasses aspects and embodiments described below.
1. A handling device for easily polymerizable substance including:
   a flow unit forming a flow path through which an easily polymerizable substance-containing material flows and in which a support member with which irregular packing can be packed is installed;
   a protrusion unit provided on a side of a packed bed packed with the irregular packing in the flow unit so as to protrude outward in a direction intersecting a flow direction of the easily polymerizable substance-containing material, and forming an internal space in a protruding site and an opening region that leads to the internal space and on which a lid that separates the internal space from the outside can be installed;
   a spray unit that can be disposed in the internal space of the protrusion unit and can spray liquid onto an inner wall surface of the protrusion unit with the spray unit disposed in the internal space; and
   a grid disposed at a boundary of the flow path and the internal space to prevent the irregular packing packed in the packed bed from moving to the internal space.
2. The handling device for easily polymerizable substance according to the above-described 1., in which meshes of the grid are formed to have a size equal to or smaller than a size of the irregular packing.
3. The handling device for easily polymerizable substance according to the above-described 1. or 2., in which the spray unit sprays liquid upward in the internal space of the protrusion unit.
4. The handling device for easily polymerizable substance according to any one of the above-described 1. to 3., in which the grid is detachable from the flow unit.
5. The handling device for easily polymerizable substance according to any one of the above-described 1. to 4., in which the spray unit can be inserted into the internal space through the lid that closes the opening region from the outside.

The present application is based on Japanese Patent Application No. 2022-073001, filed on April 27, 2022, the disclosure content of which is hereby incorporated by reference herein in its entirety.

### Reference Signs List

- 10:: flow unit
- 11:: flow path
- 17:: packed bed
- 20:: support member
- 21:: hole
- 22:: packing restraint
- 23:: liquid distributor
- 30:: protrusion unit
- 31:: internal space
- 32:: opening region
- 40, 40a, 40b, 40c, 40d:: spray unit
- 50:: grid
- F:: irregular packing
- L:: lid

## Claims

1. A handling device for easily polymerizable substance comprising:
a flow unit forming a flow path through which an easily polymerizable substance-containing material flows and in which a support member with which irregular packing can be packed is installed;
a protrusion unit provided on a side of a packed bed packed with the irregular packing in the flow unit so as to protrude outward in a direction intersecting a flow direction of the easily polymerizable substance-containing material, and forming an internal space in a protruding site and an opening region that leads to the internal space and on which a lid that separates the internal space from an outside can be installed;
a spray unit that can be disposed in the internal space of the protrusion unit and can spray liquid onto an inner wall surface of the protrusion unit with the spray unit disposed in the internal space; and
a grid disposed at a boundary of the flow path and the internal space to prevent the irregular packing packed in the packed bed from moving to the internal space.

2. The handling device for easily polymerizable substance according to claim 1, wherein meshes of the grid are configured to have a size equal to or smaller than a size of the irregular packing.

3. The handling device for easily polymerizable substance according to claim 1 or 2, wherein the spray unit sprays liquid upward in the internal space of the protrusion unit.

4. The handling device for easily polymerizable substance according to claim 1 or 2, wherein the grid is detachable from the flow unit.

5. The handling device for easily polymerizable substance according to claim 1 or 2, wherein the spray unit can be inserted into the internal space through the lid that closes the opening region from the outside.
